# EUROPEAN PATENT APPLICATION

(11) **EP 4 700 002 A1**
(43) Date of publication of application: **25.02.2026**
(21) Application number: 24306381.5
(22) Date of filing: 20.08.2024
(51) Int. Cl.: C07C 4/22, C07C 7/00, C07C 7/04, C07C 15/46

(54) **RECOVERING STYRENE FROM THE HEAVIES RESIDUE PRODUCED IN POLYSTYRENE PYROLYSIS**

(71) Applicant: Technip Energies France, 92741 Nanterre Cedex (FR)
(72) Inventor: Vallieres, Peter, 92741 Nanterre Cedex (FR); Bishop, Jonathan, 92741 Nanterre Cedex (FR); Hubbell, Douglas Stuart, 92741 Nanterre Cedex (FR)
(74) Representative: Edson, Russell Gregory

(57) **Abstract**

A system for recovering styrene after depolymerization of styrenic waste plastics may include a depolymerization reactor unit capable of generating a pyrolysis oil stream and a residue stream from a waste plastics stream; a flash drum downstream from the depolymerization reactor unit configured to receive the residue stream and to produce (i) an overhead stream comprising a higher concentration of styrene than the residue stream and (ii) a bottoms stream comprising a lower concentration of styrene than the residue stream. Optionally, the system may include a purification unit configured to receive the pyrolysis oil stream, the overhead stream, or a stream comprising a mixture of the pyrolysis oil stream and the overhead stream and to produce a styrene monomer product stream.

## Description

### Technical Field

The present disclosure relates generally to chemical processing and, more particularly (although not necessarily exclusively), to recovering additional styrene from a polystyrene depolymerization process such as a pyrolysis process using a flash drum.

### Background

Environmental pollution, resource scarcity, climate change, and the like are contemporary problems that have been driving development of cyclical, or circular, economies to replace various linear economies. For example, processes can be used for recovering raw materials from plastic waste. Plastic waste is increasing and has various negative effects on the environment, on public health, on the economy, and the like. Not all thermoplastic polymers can be recycled at similar rates or efficiencies. Tailoring a chemical recycling process to specific polymers or classes of polymers can be difficult.

### Summary

One or more embodiments include the system comprising: a depolymerization reaction unit capable of generating a pyrolysis oil stream and a residue stream from a waste plastics stream, wherein the residue stream comprises styrene; and a flash drum downstream from the depolymerization reaction unit configured to receive the residue stream and to produce (i) an overhead stream comprising a higher concentration of styrene than the residue stream and (ii) a bottoms stream comprising a lower concentration of styrene than the residue stream.

One or more embodiments include the system of any previous paragraph, further comprising: a purification unit configured to receive the pyrolysis oil stream, the overhead stream, or a stream comprising a mixture of the pyrolysis oil stream and the overhead stream, and to produce a styrene monomer product stream.

One or more embodiments include the system of any previous paragraph, wherein there is a purification unit that comprises one or more distillation columns.

One or more embodiments include the system of any previous paragraph, wherein the system is capable of recovering at least 75 wt% of the styrene from the residue stream, based on a total weight of the styrene in the residue stream.

One or more embodiments include the system of any previous paragraph, further comprising: a heat exchanger between the depolymerization reactor unit and the flash drum configured to receive and heat the residue stream.

One or more embodiments include the system of any previous paragraph, wherein the flash drum is configured to operate at a temperature ranging from 120 °C to 200 °C.

One or more embodiments include the system of any previous paragraph, wherein the flash drum is configured to operate at a vacuum pressure.

One or more embodiments include a method comprising: feeding a residue stream from a depolymerization process of a waste plastics stream to a flash drum, wherein the residue stream comprises styrene; and flashing the residue stream in the flash drum to produce (i) an overhead stream comprising a higher concentration of styrene than the residue stream and (ii) a bottoms stream comprising a lower concentration of styrene than the residue stream.

One or more embodiments include the method of any previous paragraph, further comprising: purifying the overhead stream from the flash drum and a pyrolysis oil stream from the depolymerization process to produce a plurality of streams including a styrene monomer product stream.

One or more embodiments include the method of any previous paragraph, wherein at least 75 wt% of the styrene in the residue stream is recovered, based on a total weight of the styrene in the residue stream.

One or more embodiments include the method of any previous paragraph, further comprising: preheating the residue stream before being fed to the flash drum.

One or more embodiments include the method of any previous paragraph, further comprising: mixing the overhead stream from the flash drum and the pyrolysis oil stream from the depolymerization process before purification.

One or more embodiments include the method of any previous paragraph, wherein the residue stream comprises styrene at 20 wt% or less, based on a total weight of the residue stream.

One or more embodiments include the method of any previous paragraph, wherein the waste plastics stream comprises at least 75 wt% styrenic polymer, based on a total weight of the plastics stream.

One or more embodiments include the method of any previous paragraph, wherein the waste plastics stream comprises at least 75 wt% polystyrene, based on a total weight of the plastics stream.

One or more embodiments include the method of any previous paragraph, wherein the pyrolysis oil stream comprises 50 wt% to 80 wt% styrene, based on a total weight of the pyrolysis oil stream.

One or more embodiments include the method of any previous paragraph, wherein the overhead stream comprises 40 wt% to 80 wt% styrene, based on a total weight of the overhead stream.

One or more embodiments include the method of any previous paragraph, wherein the bottoms stream comprises styrene at 10 wt% or less, based on a total weight of the bottoms stream.

One or more embodiments include the method of any previous paragraph, wherein the styrene monomer stream comprises at least 99 wt% styrene, based on a total weight of the styrene monomer stream.

One or more embodiments include the method of any previous paragraph, wherein the flash drum is operated at a temperature ranging from 120 °C to 200 °C.

One or more embodiments include the method of any previous paragraph, wherein the flash drum is operated at a vacuum pressure.

### Brief Description of the Drawings

FIG. 1 is a diagram of a styrene processing system for generating a styrene monomer product stream according to some examples of the present disclosure.
FIG. 2 is a flowchart of a process for recovering additional styrene from a depolymerization process using a flash drum according to some examples of the present disclosure.

### Detailed Description

Certain aspects and features of the present disclosure relate to recovering styrene (also referred to as "styrene monomer") from outputs from a depolymerization process using a flash drum. The depolymerization process may include a pyrolysis process or other suitable depolymerization process that produces pyrolysis oil from waste plastics, such as styrenic waste. The pyrolysis oil may include the majority of the styrene produced in the depolymerization process and, also, contaminants that may be removed via suitable purification processes to yield a styrene monomer product. The depolymerization process may also produce a residue (also referred to as "heavies residue") that may include non-volatile materials, such as heavy hydrocarbons. Unfortunately, the residue may also include as much as 30 wt% styrene. In current depolymerization system designs, styrene is not recovered from such residue. Rather, the residue is typically used as a fuel source. Since the residue may include a high concentration of styrene, a significant portion of the valuable monomer may be simply lost as waste or used as fuel in the current systems. In contrast, the present application processes the residue to extract at least some of the styrene therein.

More specifically, the methods and systems of the present disclosure include a flash drum to facilitate an adiabatic flash of the residue, which can cause at least a portion of the styrene in the residue to vaporize and separate from heavier hydrocarbons in the residue. The recovered styrene can then be processed with the pyrolysis oil for further purification to generate a styrene monomer product. Advantageously, the inclusion of the flash drum may allow for recovering over 75 wt% of the styrene from the residue. Further, because the process uses an adiabatic flash to extract styrene from the residue, the incorporation of the flash drum may be straightforward and less costly than incorporating other separation technologies like distillation for extracting styrene from the residue.

The illustrative examples herein are given to introduce the reader to the general subject matter discussed here and are not intended to limit the scope of the disclosed concepts. The following sections describe various additional features and examples with reference to the drawings in which like numerals indicate like elements but, like the illustrative examples, should not be used to limit the present disclosure.

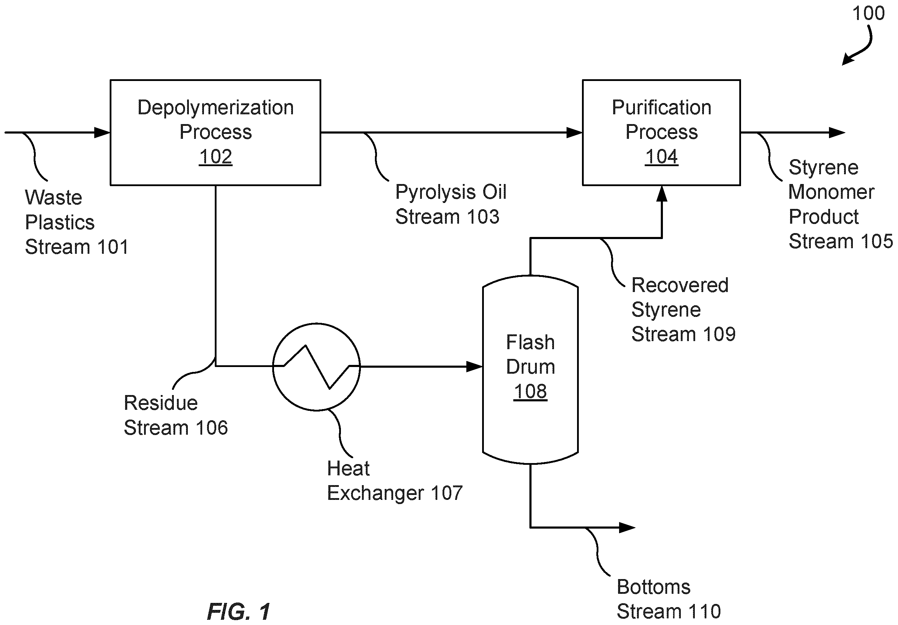

FIG. 1 illustrates a process flow diagram of a styrene processing system 100 for generating a styrene monomer product stream 105 according to at least some examples of the present disclosure. The styrene processing system 100 can be used to generate the styrene monomer product stream 105 using one or more operations or processes, such as depolymerization, purification, and the like. The styrene processing system 100 may advantageously include a flash drum 108 for extracting at least some of the styrene from a residue stream 106 to generate a greater amount of the styrene monomer product stream 105 from a waste plastics stream 101 than systems that exclude the flash drum 108.

As illustrated in FIG. 1, the waste plastics stream 101 may be a feed for a depolymerization process 102. The depolymerization of the waste plastics stream 101 may be performed using pyrolysis (e.g., in a pyrolysis reactor) to convert the waste plastics stream 101 into a plurality of streams including a pyrolysis oil stream 103 and a residue stream 106.

The waste plastics stream 101 may have a styrenic polymer content of at least 75 wt% (e.g., 75 wt% to 100 wt%, 80 wt% to 100 wt%, or 90 wt% to 100 wt%), based on a total weight of the waste plastics stream 101. The waste plastics stream 101 may have a polystyrene content of at least 75 wt% (e.g., 75 wt% to 100 wt%, 80 wt% to 100 wt%, or 90 wt% to 100 wt%), based on a total weight of the waste plastics stream 101.

The depolymerization process 102 may include depolymerizing waste plastics containing styrenic polymers in a pyrolysis reactor.

The pyrolysis oil stream 103 may be the main product of the depolymerization process. The pyrolysis oil stream 103 may contain most of the styrene produced in the depolymerization process. The pyrolysis oil stream 103 may include styrene at a concentration ranging from 50 wt% to 80 wt% (e.g., 50 wt% to 65 wt%, 55 wt% to 75 wt%, or 60 wt% to 80 wt%). The pyrolysis oil may further contain compounds that may include, but are not limited to, benzene, toluene, ethylbenzene, alpha-methylstyrene, cumene, xylenes, compounds lighter than benzene, compounds heavier than alpha-methylstyrene, and any combination thereof.

The residue stream 106 may include heavy, non-volatile material and styrene. In some examples, the residue stream 106 may include styrene at a concentration of 20 wt% or less (e.g., greater than 0 wt% to 20 wt%, 1 wt% to 20 wt%, 5 wt% to 20 wt%, or 10 wt% to 20 wt%), based on a total weight of the residue stream 106. The residue stream 106 may contain concentrations of styrene greater than 20 wt%. The remainder of the residue stream 106 may include low-volatility materials like heavy hydrocarbons such as polycyclic aromatic compounds.

The residue stream 106 may be conveyed to the flash drum 108 to extract at least a portion of the styrene from the residue stream. The flash drum 108 may receive the residue stream 106 and may cause the residue stream 106 to undergo an adiabatic flash, which may result in vaporization of at least a portion of the residue stream 106 and produce an overhead stream 109. The remaining portion of the residue stream 106 may produce the bottoms stream 110.

The flash drum 108 may operate at a temperature ranging from 120 °C to 200 °C (e.g., 120 °C to 180 °C, 120 °C to 160 °C, or 135 °C to 160 °C). If needed, a polymerization inhibitor may be included at higher operating temperatures of the flash drum. The flash drum 108 may operate at a vacuum pressure (i.e., a pressure below atmospheric pressure). The operating pressure may be primarily dependent on the upstream system pressure. In some instances, the flash drum 108 may operate at 700 mm Hg or below (e.g., 65 mm Hg to 700 mm Hg, 65 mm Hg to 600 mm Hg, or 65 mm Hg to 400 mm Hg). In some instances, the pressure in the flash drum 108 may be controlled or otherwise influenced by downstream pressures. For example, the flash drum may be operated in a pressure range between the pyrolysis reactor in the depolymerization process 102 and a distillation column in the purification process 104. In one example, the depolymerization process 102 may include a flash to generate the pyrolysis oil stream 103 and the residue stream 106, and therefore, the pressure of the flash drum 108 should be lower to ensure the production of styrene-rich vapor in an adiabatic flash. In another example, the overhead stream 109 (or recovered styrene stream) from the flash drum may be routed directly to a distillation column in the purification process 104. In such instances, operating the flash drum 108 at a higher pressure than the distillation column may mitigate or eliminate the need for additional equipment such as a compressor to convey the overhead stream 109 to a distillation column of the purification process 104.

In some examples, the recovery of styrene from the flash drum 108 may be improved when the residue stream 106 is preheated before entering the flash drum 108. Accordingly, the styrene processing system 100 may include a heat exchanger 107 positioned upstream from the flash drum 108 to heat the residue stream 106. Any suitable heating medium (e.g., steam) may be used in the heat exchanger to provide heat to the residue stream 106. The residue stream 106 may be preheated to at least 10°C (e.g., 10°C to 50°C, 20°C to 45°C, or 20°C to 40°C) below the operating temperature.

The overhead stream 109 from the flash drum 108 may include styrene at a higher concentration than the concentration of styrene in the residue stream 106. The overhead stream 109 may include styrene at a concentration ranging from 40 wt% to 80 wt% (e.g., 45 wt% to 80 wt%, 50 wt% to 80 wt%, 60 wt% to 80 wt%, 65 wt% to 80 wt%, or 50 wt% to 70 wt%), based on a total weight of the overhead stream 109. In some examples, the overhead stream 109 may additionally include other volatile materials vaporized from the residue stream 106 in the flash drum 108.

The bottoms stream 110 from the flash drum 108 may include contaminants, heavy hydrocarbons, non-volatile materials, or any combination thereof separated from the residue stream 106. Styrene may be present in the bottoms stream 110 at a concentration of 10 wt% or less (e.g., 0 wt% to 10 wt%, 0.01 wt% to 10 wt%, 0.01 wt% to 7 wt%, 0 wt% to 7 wt%, 0.1 wt% to 5 wt%, 0.01 wt% to 5 wt%, or 0 wt% to 5 wt%), based on a total weight of the bottoms stream 110. The bottoms stream 110 may be used in downstream processes or may be used as fuel.

The overhead stream 109 and the pyrolysis oil stream 103 may be purified in the purification process 104 to produce a styrene monomer product stream 105. In some examples, the purification process 104 may use one or more distillation columns, one or more stripping columns, other purification processes, or any combination thereof to purify the overhead stream 109 and the pyrolysis oil stream 103. For example, distillation of styrene may typically be operated under significantly low vacuum pressure to increase the relative volatility of the compounds being separated, which may make the separation of styrene from other compounds easier and allow for a low temperature in the distillation columns to mitigate unwanted polymer formation.

In some examples, the overhead stream 109 may be combined with the pyrolysis oil stream 103 as feed to the purification process 104. In other examples, the overhead stream 109 may be fed to the purification process 104 separately from the pyrolysis oil stream 103. The overhead stream 109 may be co-fed to the same purification step in the purification process 104, or alternatively, the overhead stream 109 may be fed to a different purification step than the pyrolysis oil stream 103 depending on the composition of each of the streams. Alternatively, the purification process may be conducted and have the corresponding equipment at a different location than the other portions of the styrene processing system 100.

The styrene monomer product stream 105 may include styrene at a concentration of at least 99 wt% (e.g., 99 wt% to 100 wt%, 99.5 wt% to 100 wt%, 99.8 wt% to 100 wt%, 99.85 wt% to 100 wt% or 99.9 wt% to 100 wt%), based on a total weight of the styrene monomer product stream 105.

Accordingly, a system of the present disclosure may include a depolymerization reactor unit capable of generating a pyrolysis oil stream and a residue stream from a waste plastics stream, wherein the residue stream comprises styrene; a flash drum downstream from the depolymerization reactor unit configured to receive the residue stream and to produce (i) an overhead stream comprising a higher concentration of styrene than the residue stream and (ii) a bottoms stream comprising a lower concentration of styrene than the residue stream; and, optionally, a purification unit configured to receive the pyrolysis oil stream, the overhead stream, or a stream comprising a mixture of the pyrolysis oil stream and the overhead stream and to produce a styrene monomer product stream. The system may further include a heat exchanger between the depolymerization reactor unit and the flash drum configured to receive and heat the residue stream.

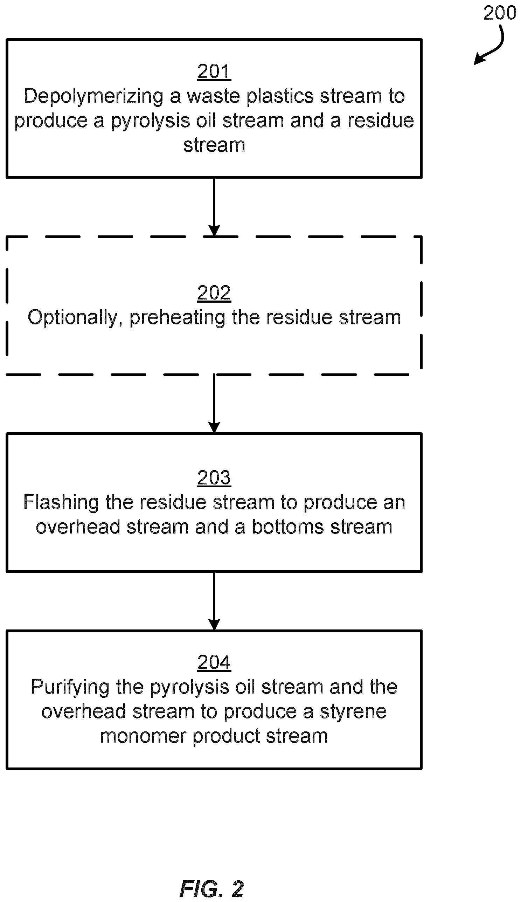

FIG. 2 illustrates a flow chart for a method 200 for producing a styrene monomer product from a residue stream produced in a depolymerization process. The operating conditions, stream compositions, and the like described relative to FIG. 1 apply to the methods of the present disclosure including the example method of FIG. 2.

Step 201 may include depolymerizing a waste plastics stream, which may contain styrenic polymers (e.g., in a depolymerization process that includes a pyrolysis reactor) and the step may produce a plurality of streams including a pyrolysis oil stream and a residue stream. Step 202 may optionally be performed and includes preheating the residue stream (e.g., in a heat exchanger). Step 203 may include flashing (e.g., adiabatically flashing) (e.g., in a flash drum) the residue stream to produce an overhead stream and a bottoms stream. Step 204 may include purifying the pyrolysis oil stream and the overhead stream (e.g., in a purification process like distillation, stripping, or a combination thereof) to produce a styrene monomer product stream. Step 204 is optional since purification may be performed at a different location than the other steps.

The systems and methods of the present disclosure may recover at least 75 wt% (e.g., at least 78 wt%, at least 80 wt%, at least 85 wt%, 75 wt% to 95 wt%, 75 wt% to 85 wt%, or 75 wt% to 70 wt%) of the styrene in the residue stream, based on a total weight of the styrene in the residue stream.

The foregoing description of certain examples, including illustrated examples, has been presented only for the purpose of illustration and description and is not intended to be exhaustive or to limit the disclosure to the precise forms disclosed. Numerous modifications, adaptations, and uses thereof will be apparent to those skilled in the art without departing from the scope of the disclosure. For instance, any examples described herein can be combined with any other examples to yield further examples.

## Claims

1. A system comprising:
a depolymerization reaction unit capable of generating a pyrolysis oil stream and a residue stream from a waste plastics stream, wherein the residue stream comprises styrene; and
a flash drum downstream from the depolymerization reaction unit configured to receive the residue stream and to produce (i) an overhead stream comprising a higher concentration of styrene than the residue stream and (ii) a bottoms stream comprising a lower concentration of styrene than the residue stream.

2. The system of claim 1 further comprising:
a purification unit configured to receive the pyrolysis oil stream, the overhead stream, or a stream comprising a mixture of the pyrolysis oil stream and the overhead stream and to produce a styrene monomer product stream.

3. The system of claim 2, wherein there is a purification unit that comprises one or more distillation columns.

4. The system of claim 1 or 2, wherein the system is capable of recovering at least 75 wt% of the styrene from the residue stream, based on a total weight of the styrene in the residue stream.

5. The system of claim 1 or 2 further comprising:
a heat exchanger between the depolymerization reactor unit and the flash drum configured to receive and heat the residue stream.

6. The system of claim 1 or 2, wherein the flash drum is configured to operate at a vacuum pressure.

7. A method comprising:
feeding a residue stream from a depolymerization process of a waste plastics stream to a flash drum, wherein the residue stream comprises styrene; and
flashing the residue stream in the flash drum to produce (i) an overhead stream comprising a higher concentration of styrene than the residue stream and (ii) a bottoms stream comprising a lower concentration of styrene than the residue stream.

8. The method of claim 7, wherein at least 75 wt% of the styrene in the residue stream is recovered, based on a total weight of the styrene in the residue stream.

9. The method of claim 7 or 8 further comprising:
purifying the overhead stream from the flash drum and a pyrolysis oil stream from the depolymerization process to produce a plurality of streams including a styrene monomer product stream.

10. The method of claim 7 or 8 further comprising:
preheating the residue stream before being fed to the flash drum.

11. The method of claim 7 or 8 further comprising:
mixing the overhead stream from the flash drum and the pyrolysis oil stream from the depolymerization process before purification.

12. The method of claim 7 or 8, wherein the residue stream comprises styrene at 20 wt% or less, based on a total weight of the residue stream.

13. The method of claim 7 or 8, wherein the waste plastics stream comprises at least 75 wt% styrenic polymer, based on a total weight of the plastics stream.

14. The method of claim 7 or 8, wherein the overhead stream comprises 40 wt% to 80 wt% styrene, based on a total weight of the overhead stream.

15. The method of claim 7 or 8, wherein the flash drum is operated at a temperature ranging from 120 °C to 200 °C.
